(19) Europäisches Patentamt
European Patent Office
Office européen des brevets

(11) Veröffentlichungsnummer : **0 035 753**
**B1**

# (12) EUROPÄISCHE PATENTSCHRIFT

(45) Veröffentlichungstag der Patentschrift : **22.06.83**

(51) Int. Cl.³ : **C 07 C 69/743**, C 07 C 17/18, C 07 C 67/307

(21) Anmeldenummer : **81101575.9**

(22) Anmeldetag : **05.03.81**

## (54) Verfahren zur Herstellung von 1,1-Dichlor-alkenen.

(30) Priorität : **12.03.80 DE 3009486**

(43) Veröffentlichungstag der Anmeldung : **16.09.81 Patentblatt 81/37**

(45) Bekanntmachung des Hinweises auf die Patenterteilung : **22.06.83 Patentblatt 83/25**

(84) Benannte Vertragsstaaten : **AT BE CH DE FR GB IT LI NL**

(56) Entgegenhaltungen : **EP A 0 002 849**

(73) Patentinhaber : **BAYER AG**
**Zentralbereich Patente, Marken und Lizenzen**
**D-5090 Leverkusen 1, Bayerwerk (DE)**

(72) Erfinder : **Riebel, Hans-Jochem, Dr.**
**In der Beek 92**
**D-5600 Wuppertal 1 (DE)**
Erfinder : **Maurer, Fritz, Dr.**
**Roeberstrasse 8**
**D-5600 Wuppertal 1 (DE)**
Erfinder : **Priesnitz, Uwe, Dr.**
**Severinstrasse 58**
**D-5650 Solingen 1 (DE)**

**EP 0 035 753 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von 1,1-Dichlor-alkenen

Die Erfindung betrifft ein neues Verfahren zur Herstellung von 1,1-Dichloralkenen.

Es ist bekannt, daß man 1,1-Dichlor-alkene erhält, wenn man Lithiumsalze von Dichlormethanphosphonsäureestern mit Aldehyden oder Ketonen umsetzt (vergleiche Synthesis *1975,* 458-461 und 535-536). Die Herstellung der Lithiumsalze von Dichlormethanphosphonsäureestern ist jedoch aufwendig. Man erhält sie aus Chlormethanphosphonsäureestern bzw. Trichlormethanphosphonsäureestern durch Umsetzung mit Butyllithium und gegebenenfalls Tetrachlorkohlenstoff bei − 70 bis − 80 °C, wobei sorgfältig getrocknete Lösungsmittel zu verwenden sind und eine Inertgasatmosphäre erforderlich ist.

Aus EP-A-2849 ist bekannt, daß man bestimmte 1,1-Dihalogenalkene erhält, indem man das Reaktionsprodukt aus tris-(Dialkylamino)phosphin und Tetrahalogenmethan oder Trihalogenmethan mit einem Aldehyd umsetzt. Das Reaktionsprodukt aus tris-(Dialkylamino)phosphin und Tetrahalogenmethan oder Trihalogenmethan ist ein Ylid zu dessen Bildung 2 Mol des teuren Phosphins pro Mol Halogenmethan erforderlich sind.

Es wurde nun ein Verfahren zur Herstellung von 1,1-Dichlor-alkenen der Formel I

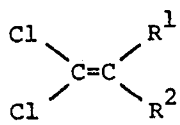

(I)

gefunden in welcher

$R^1$ für Wasserstoff, für gegebenenfalls halogen-substituiertes $C_1$-$C_5$-Alkyl, für gegebenenfalls halogen-substituiertes Benzyl oder Phenylethyl, oder für gegebenenfalls durch Halogen, $C_1$-$C_4$-Alkyl, $C_1$-$C_4$-Alkoxy, Methylendioxy, Cyano und/oder Nitro substituiertes Phenyl, und

$R^2$ für gegebenenfalls halogen-substituiertes $C_1$-$C_5$-Alkyl, für $C_2$-$C_5$-Alkenyl, für $C_2$-$C_5$-Alkinyl, für gegebenenfalls halogen-substituiertes Benzyl oder Phenylethyl, für gegebenenfalls durch Halogen, $C_1$-$C_4$-Alkyl, $C_1$-$C_4$-Alkoxy, Methylendioxy, Cyano und/oder Nitro substituiertes Phenyl, für gegebenenfalls halogen-substituiertes Styryl oder für den Rest

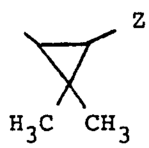

worin Z für Acetyl, Cyano, Carbamoyl, $C_1$-$C_4$-Alkoxycarbonyl oder für COOM steht, wobei M für Wasserstoff, ein Alkalimetall, ein Erdalkalimetalläquivalent oder einen Ammoniumrest steht,
das dadurch gekennzeichnet ist, daß man Carbonylverbindungen der Formel II

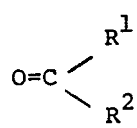

(II)

in welcher $R^1$ und $R^2$ die oben angegebene Bedeutung haben, mit Trichlormethanphosphonsäureestern der Formel III

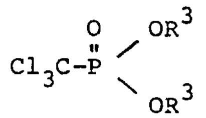

(III)

in welcher $R^3$ einzeln für Alkyl oder Phenyl steht oder beide Reste $R^3$ zusammen für Alkandiyl (Alkylen) stehen, in Gegenwart von wenigstens der äquimolaren Menge eines Phosphorigsäuretriamids der Formel IV

$$P(NR^4{}_2)_3 \qquad \text{(IV)}$$

in welcher $R^4$ für Alkyl steht oder beide Reste $R^4$ zusammen für Alkandiyl (Alkylen) stehen, und gegebenenfalls in Gegenwart eines Verdünnungsmittels bei Temperaturen zwischen 0 und 150 °C umsetzt.

Es ist als überraschend anzusehen, daß man nach dem erfindungsgemäßen Verfahren 1,1-Dichloralkene der Formel (I) wesentlich einfacher und kostengünstiger in guten Ausbeuten erhält als man in Anbetracht des Standes der Technik erwarten konnte.

So ist pro Mol Phosphonsäureester nur etwa 1 Mol tris-(Dialkylamino)phosphin notwendig. Die Herstellung der 1,1-Dihalogenalkane läßt sich also kostengünstiger gestalten als es aus dem nach EP-A-2849 bekannten Stand der Technik zu erwarten gewesen wäre.

Es konnte auch nicht erwartet werden, daß die erfindungsgemäße Reaktion überhaupt abläuft. Zwar war bekannt gewesen, daß zu Olefinierungsreaktionen Lithiumsalze von Dichlormethanphosphonsäureestern eingesetzt werden können (Synthesis 1975, S. 535 ff.) doch konnte man nicht erwarten, daß Salz-Komplexe wie sie vermutlich bei der erfindungsgemäßen Reaktion entstehen ebenfalls zur Olefinierung geeignet sind.

Verwendet man als Ausgangsstoffe beispielsweise 3-Formyl-2,2-dimethyl-cyclopropan-1-carbonsäuremethylester, Trichlormethanphosphonsäuredimethylester und Phosphorigsäure-tris-dimethylamid, so kann die Reaktion dieser Verbindungen durch folgendes Formelschema skizziert werden :

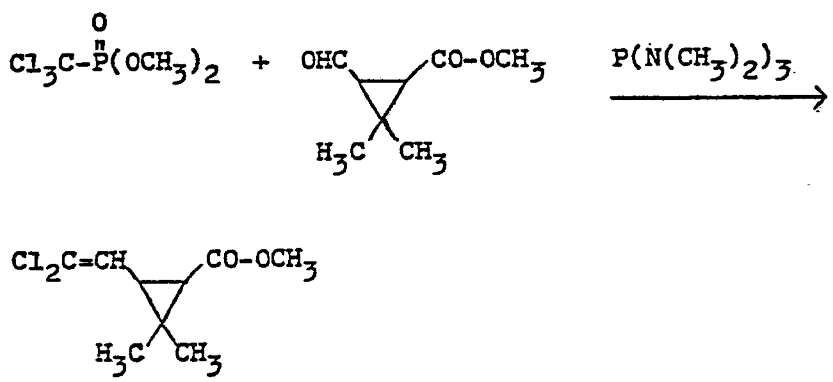


Als Ausgangsstoffe besonders bevorzugt sind diejenigen Verbindungen der Formel (II), in welcher
$R^1$ für Wasserstoff steht und
$R^2$ für $C_2$-$C_5$-Alkenyl oder den Rest

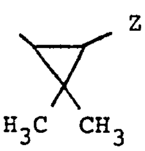

steht, worin Z für Cyano, Acetyl, Carboxy, $C_{1-4}$-Alkoxycarbonyl oder für COOM steht, wobei M für Natrium oder Kalium steht.

Durch Hydrolyse von bekannten Cyclopropancarbonsäureestern der Formel (VIII) (vergleiche J. Org. Chem. *32* (1967), 3351-3355 ; Bull. Soc. Chim. Belg. *87* (1978), 721-732 ; Tetrahedron Lett. *1978,* 1847-1850), beispielsweise durch Umsetzung mit wässrig-alkoholischer Kalilauge bei Temperaturen zwischen 20 und 100 °C und anschließendes Ansäuern erhält man die Carbonsäuren der Formel (VII). Diese können durch Umsetzung mit Halogenierungsmitteln, wie z. B. Thionylchlorid, bei Temperaturen zwischen 20 und 80 °C in die Säurechloride der Formel (VI) umgewandelt werden.

Durch Umsetzung der Säurechloride (VI) mit Trialkylphosphiten bei Temperaturen zwischen − 20 und + 150 °C, vorzugsweise zwischen 0 und 120 °C, erhält man die Cyclopropanoylphosphonsäureester der Formel (V) (vergleiche J. Am. Chem. Soc. *86* (1964), 3862-3866 ; Methoden der organischen Chemie (Houben-Weyl-Müller), 4. Auflage, Band 12/1, S. 453, Georg-Thieme-Verlag, Stuttgart 1963). Zur Isolierung und Reinigung der Produkte wird, gegebenenfalls unter vermindertem Druck destilliert.

Die α-Hydroxy-phosphonsäureester der Formel (IV) erhält man durch Reduktion der Oxoverbindungen der Formel (V) mit Natriumtetrahydridoborat, gegebenenfalls unter Verwendung eines Verdünnungsmittels, wie z. B. Wasser oder wässriges Methanol, bei Temperaturen zwischen − 20 und + 50 °C und Halten des pH-Wertes zwischen 5 und 8 durch Zugabe eines Puffermittels, wie z. B. Natriumhydrogenphosphat (vergleiche Chem. Ber. *103* (1970), 2984-2986). Zur Aufarbeitung extrahiert man mit einem mit Wasser nicht mischbaren Lösungsmittel, wie z. B. Methylenchlorid, trocknet die Extrakte, filtriert und destilliert das Lösungsmittel unter vermindertem Druck ab.

Aus den α-Hydroxy-phosphonsäureestern der Formel (IV) können die entsprechenden Aldehyde der Formel (IIa) durch Behandeln mit Natronlauge bei Temperaturen zwischen 0 und 100 °C, vorzugsweise zwischen 10 und 50 °C hergestellt werden (vergleiche Chem. Ber. *103* (1970), 2984-2986).

Alternativ zum oben skizzierten Herstellungsverfahren erhält man Aldehyde der Formel (IIa) auch

durch Umsetzung von Säurechloriden der Formel (VI) mit Lithiumtritert.-butoxy-hydrido-aluminat, welches man gegebenenfalls *in situ* aus Lithium-tetrahydridoaluminat und tert.-Butanol hergestellt hat, gegebenenfalls in Gegenwart eines Verdünnungsmittels, wie z. B. Tetrahydrofuran, bei Temperaturen zwischen − 100 und + 100 °C, vorzugsweise zwischen − 80 und + 50 °C. Zur Aufarbeitung wird in eine Mischung aus Salzsäure und Eiswasser gegossen und mit einem mit Wasser nicht mischbaren Lösungsmittel, wie z. B. Diethylether, extrahiert. Die Extrakte werden getrocknet, filtriert und eingeengt. Zur Reinigung des Rohproduktes wird gegebenenfalls destilliert.

Die weiter als Ausgangsstoffe zu verwendenden Trichlormethanphosphonsäureester sind durch Formel (III) definiert. Vorzugsweise stehen darin die Reste

$R^3$ einzeln für $C_1$-$C_4$-Alkyl oder Phenyl oder beide Reste $R^3$ stehen zusammen für $C_2$-$C_5$-Alkandiyl.

Als Beispiele seien Trichlormethanphosphonsäuredimethylester, -diethylester-, -dipropylester und -diphenylester genannt.

Verbindungen der Formel (III) sind bekannt (vergleiche J. Am. Chem. Soc. *69* (1947), 1022 ; ibid. *77* (1955), 1156).

Die weiter beim erfindungsgemäßen Verfahren einzusetzenden Phosphorigsäuretriamide sind durch Formel (IV) definiert. Vorzugsweise steht darin

$R^4$ für $C_1$-$C_4$-Alkyl oder beide Reste $R^4$ stehen zusammen für $C_2$-$C_5$-Alkandiyl.

Als Beispiele seien Phosphorigsäure-tris-dimethylamid und -tris-diethylamid genannt.

Das erfindungsgemäße Verfahren wird gegebenenfalls unter Verwendung von Verdünnungsmitteln durchgeführt. Als solche kommen praktisch alle inerten organischen Lösungsmittel insbesondere aprotisch polare Solventien in Frage. Hierzu gehören Ether, wie z. B. Glycoldimethylether, Diglycoldimethylether, Tetrahydrofuran und Dioxan, Carbonsäureamide, wie z. B. Dimethylformamid, Dimethylacetamid und N-Methylpyrrolidon, Sulfoxide, wie z. B. Dimethylsulfoxid, Phosphorsäureamide, wie z. B. Hexamethylphosphorsäuretriamid, sowie Nitrile, wie z. B. Acetonitril und Propionitril.

Die Reaktionstemperatur kann innerhalb eines größeren Bereichs variiert werden. Im allgemeinen arbeitet man zwischen 0 und 150 °C, vorzugsweise bei 10 bis 100 °C.

Das erfindungsgemäße Verfahren wird im allgemeinen bei Normaldruck durchgeführt.

Je Mol Carbonylverbindung der Formel (II) werden 0,9 bis 1,2 Mol, vorzugsweise 0,95 bis 1,1 Mol Trichlormethanphosphonsäureester der Formel (III) und 0,9 bis 1,2 Mol, vorzugsweise 0,95 bis 1,1 Mol eines Phosphorigsäuretriamids der Formel (IV) eingesetzt.

Zur Durchführung des erfindungsgemäßen Verfahrens werden vorzugsweise die Ausgangsstoffe der Formeln (II) und (III) vorgelegt und das Phosphorigsäuretriamid zugetropft. Das Reaktionsgemisch wird bis zum Ende der Umsetzung gerührt und dann filtriert.

Die Aufarbeitung kann nach üblichen Methoden durchgeführt werden, beispielsweise, indem man das Filtrat mit Wasser verdünnt, mit einem mit Wasser praktisch nicht mischbaren, organischen Lösungsmittel, wie z. B. Methylenchlorid, extrahiert, die organische Phase trocknet, filtriert und eindampft. Die im Rückstand verbleibenden Produkte können auf übliche Weise, z. B. durch Vakuumdestillation, gereinigt werden.

Die nach dem erfindungsgemäßen Verfahren herzustellenden 1,1-Dichlor-alkene können zum Teil als Zwischenprodukte zur Herstellung von insektizid und akarizid wirksamen Pyrethroiden verwendet werden (vergleiche DE-OS 2 326 077).

## Herstellungsbeispiele

### Beispiel 1

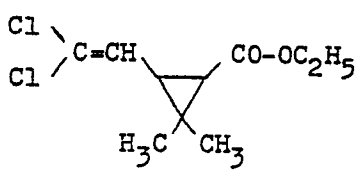

Zu einer Mischung von 25,5 g (0,1 Mol) Trichlormethanphosphonsäurediethylester und 17,2 g (0,1 Mol) 3-Formyl-2,2-dimethyl-cyclopropan-1-carbonsäureethylester werden 16,3 g (0,1 Mol) Tris-dimethylaminophosphin bei einer Temperatur von 40 °C getropft. Danach wird 3 bis 4 Stunden auf 90 °C erwärmt. Anschließend wird abgekühlt und das Reaktionsgemisch in 200 ml Wasser gegossen. Diese Suspension wird 2 mal mit je 100 ml Methylenchlorid extrahiert. Die vereinigten Extrakte werden über Natriumsulfat getrocknet und dann eingeengt. Man erhält 3-(2,2-Dichlor-vinyl)-2,2-dimethyl-cyclopropan-1-carbonsäureethylester in einer Ausbeute von 88% der Theorie.

**Anspruch**

Verfahren zur Herstellung von 1,1-Dichlor-alkenen der Formel I

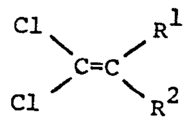

(I)

in welcher

$R^1$ für Wasserstoff, für gegebenenfalls halogen-substituiertes $C_1$-$C_5$-Alkyl, für gegebenenfalls halogensubstituiertes Benzyl oder Phenylethyl, oder für gegebenenfalls durch Halogen, $C_1$-$C_4$-Alkyl, $C_1$-$C_4$-Alkoxy, Methylendioxy, Cyano und/oder Nitro substituiertes Phenyl, und

$R^2$ für gegebenenfalls halogen-substituiertes $C_1$-$C_5$-Alkyl, für $C_2$-$C_5$-Alkenyl, für $C_2$-$C_5$-Alkinyl, für gegebenenfalls halogen-substituiertes Benzyl oder Phenylethyl, für gegebenenfalls durch Halogen, $C_1$-$C_4$-Alkyl, $C_1$-$C_4$-Alkoxy, Methylendioxy, Cyano und/oder Nitro substituiertes Phenyl, für gegebenenfalls halogen-substituiertes Styryl oder für den Rest

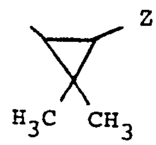

worin Z für Acetyl, Cyano, Carbamoyl, $C_1$-$C_4$-Alkoxycarbonyl oder für COOM steht, wobei M für Wasserstoff, ein Alkalimetall, ein Erdalkalimetalläquivalent oder einen Ammoniumrest steht, dadurch gekennzeichnet, daß man Carbonylverbindungen der Formel II

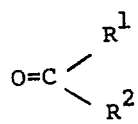

(II)

in welcher $R^1$ und $R^2$ die oben angegebene Bedeutung haben, mit Trichlormethanphosphonsäureestern der Formel III

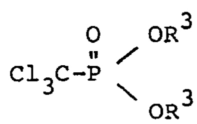

in welcher $R^3$ einzeln für Alkyl oder Phenyl steht oder beide Reste $R^3$ zusammen für Alkandiyl (Alkylen) stehen, in Gegenwart von wenigstens der äquimolaren Menge eines Phosphorigsäuretriamids der Formel IV

$$P(NR^4_2)_3 \quad (IV)$$

in welcher $R^4$ für Alkyl steht oder beide Reste $R^4$ zusammen für Alkandiyl (Alkylen) stehen, und gegebenenfalls in Gegenwart eines Verdünnungsmittels bei Temperaturen zwischen 0 und 150 °C umsetzt.

**Claim**

Process for the preparation of 1,1-dichloroalkenes of the formula I

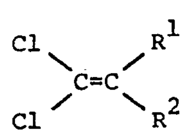

(I)

in which

$R^1$ represents hydrogen, optionally halogensubstituted $C_1$-$C_5$-alkyl, optionally halogensubstituted benzyl or phenylethyl, or phenyl which is optionally substituted by halogen, $C_1$-$C_4$-alkyl, $C_1$-$C_4$-alkoxy, methylenedioxy, cyano and/or nitro, and

$R^2$ represents optionally halogen-substituted $C_1$-$C_5$-alkyl, $C_2$-$C_5$-alkenyl, $C_2$-$C_5$-alkinyl, optionally halogen-substituted benzyl or phenylethyl, phenyl which is optionally substituted by halogen, $C_1$-$C_4$-

alkyl, $C_1$-$C_4$-alkoxy, methylenedioxy, cyano and/or nitro, optionally halogensubstituted styryl or the radical

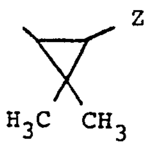

wherein Z represents acetyl, cyano, carbamoyl, $C_1$-$C_4$-alkoxycarbonyl or COOM, wherein M represents hydrogen, an alkali metal, one alkaline earth metal equivalent or an ammonium radical, characterised in that carbonyl compounds of the formula II

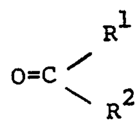 (II)

in which $R^1$ and $R^2$ have the abovementioned meaning, are reacted with trichloromethanephosphonic acid esters of the formula III

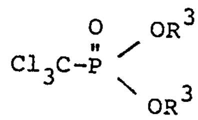

in which the two radicals $R^3$ individually represent alkyl or phenyl or the two radicals $R^3$ together represent alkanediyl (alkylene), in the presence of at least the equimolar amount of a phosphorous acid triamide of the formula IV

$$P(NR^4{}_2)_3 \quad (IV)$$

in which $R^4$ represents alkyl or the two radicals $R^4$ together represent alkanediyl (alkylene), and if appropriate in the presence of a diluent, at temperatures between 0 and 150 °C.

**Revendication**

Procédé de fabrication de 1,1-dichloroalcènes de formule I

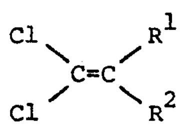 (I)

dans laquelle

$R^1$ représente de l'hydrogène, un alcoyle en $C_1$-$C_5$ éventuellement halogéno-substitué, un benzyle ou phényléthyle éventuellement halogéno-substitué ou un phényle éventuellement substitué par de l'halogène, alcoyle en $C_1$-$C_4$, alcoxy en $C_1$-$C_4$, méthylènedioxy, cyano et/ou nitro, et

$R^2$ représente un alcoyle en $C_1$-$C_5$ éventuellement halogéno-substitué, alcényle en $C_2$-$C_5$, alcinyle en $C_2$-$C_5$, benzyle ou phényléthyle éventuellement halogéno-substitué, phényle éventuellement substitué par de l'halogène, alcoyle en $C_1$-$C_4$, alcoxy en $C_1$-$C_4$, méthylènedioxy, cyano et/ou nitro, un styryle éventuellement halogéno-substitué ou le radical

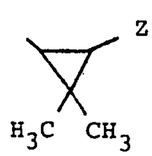

dans lequel Z représente un acétyle, cyano, carbamoyle, alcoxy ($C_1$-$C_4$) carbonyle ou COOM, M représentant de l'hydrogène, un métal alcalin, un équivalent de métal alcalino-terreux ou un radical ammonium, caractérisé en ce qu'on fait réagir des composés carbonylés de formule II

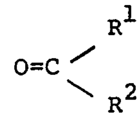

(II)

dans laquelle $R^1$ et $R^2$ ont la signification indiquée plus haut, avec des esters d'acide trichlorométhane-phosphonique de formule III

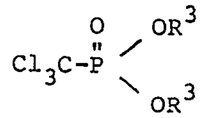

dans laquelle les $R^3$ représentent individuellement un alcoyle ou phényle ou les deux radicaux $R^3$ représentent ensemble un alcanediyle (alcoylène), en présence d'au moins la quantité équimolaire d'une triamide d'acide phosphoreux de formule IV

$$P(NR^4_2)_3 \qquad (IV)$$

dans laquelle les $R^4$ représentent un alcoyle ou les deux radicaux $R^4$ représentent ensemble un alcanediyle (alcoylène), et éventuellement en présence d'un diluant à des températures entre 0 et 150 °C.